Europäisches Patentamt

European Patent Office

Office européen des brevets

(11) Veröffentlichungsnummer: **0 261 621 B1**

# EUROPÄISCHE PATENTSCHRIFT

(45) Veröffentlichungstag der Patentschrift: **22.01.92**

(51) Int. Cl.⁵: **G01B 11/24**, A61B 5/103

(21) Anmeldenummer: **87113761.8**

(22) Anmeldetag: **21.09.87**

(54) **Vorrichtung zur Ermittlung der Abmessungen eines Gegenstandes auf photographischem Wege.**

(30) Priorität: **24.09.86 DE 3632450**

(43) Veröffentlichungstag der Anmeldung:
**30.03.88 Patentblatt 88/13**

(45) Bekanntmachung des Hinweises auf die
Patenterteilung:
**22.01.92 Patentblatt 92/04**

(84) Benannte Vertragsstaaten:
**AT BE CH FR GB LI NL SE**

(56) Entgegenhaltungen:
**DE-A- 3 301 864**
**DE-A- 3 425 913**
**DE-C- 2 948 010**
**US-A- 4 480 920**
**US-A- 4 564 295**

(73) Patentinhaber: **Landwehr, Ulrich M.**
**Bahnhofstrasse 8**
**W-3000 Hannover 1(DE)**

(72) Erfinder: **Landwehr, Ulrich M.**
**Bahnhofstrasse 8**
**W-3000 Hannover 1(DE)**

(74) Vertreter: **Döring, Roger, Dipl.-Ing.**
**Kabelkamp 20**
**W-3000 Hannover 1(DE)**

## Beschreibung

Die Erfindung betrifft eine Vorrichtung zur Ermittlung der Abmessungen eines Gegenstandes auf photographischem Wege, insbesondere der menschlichen Körpermaße, bei der mit einer Kamera eine Photographie des Gegenstandes zusammen mit einem Maßraster herstellbar ist und während der Aufnahme ein schräg von oben einfallendes Muster aus horizontal verlaufenden Linien auf den Gegenstand projizierbar ist, bei der das Maßraster mittels eines Projektors und eines das Maßraster aufweisenden Diapositivs direkt auf die Bildebene bzw. den Film projizierbar ist, bei der ein unter einem Winkel von nahezu 45° zur Bild- bzw. Filmebene geneigter lichtdurchlässiger Spiegel vorgesehen ist und bei der der Projektor auf den Spiegel ausgerichtet ist.

Aus der DE-OS 34 25 913 ist ein Verfahren bekannt, bei dem das Maßraster mittels eines vor dem Objektiv der Kamera angeordneten halbdurchlässigen Spiegels seitlich eingespiegelt wird. Bei einem Ausführungsbeispiel wird das Bild des Maßrasters direkt eingespiegelt, wobei der Abstand einer das Maßraster aufweisenden Wand zum Spiegel nahezu gleich dem Abstand des zu photographierenden Gegenstandes zum Spiegel ist. Diese Vorgehensweise hat den Nachteil, daß große Räume erforderlich sind.

Nach einem anderen Ausführungsbeispiel wird das Maßraster mittels eines Blitzprojektors und eines das Maßraster reproduzierenden Diapositivs eingespiegelt. Auch diese Vorgehensweise setzt große Räume voraus. Man kann diesen Nachteil verringern, indem man ein Objektiv mit einem großen Tiefenschärfenbereich verwendet, jedoch sind derartige Objektive in der Regel sehr teuer.

Ein beiden Lösungen gemeinsamer Nachteil liegt darin, daß durch Unachtsamkeit des Personals die exakte Justierung leicht beseitigt werden kann, was zu falschen Aussagen bei der Auswertung der Aufrahmen führt.

Aus der US-A-4 480 920 ist eine Kamera für die sogenannte Moiree-Topographie bekannt, mit deren Hilfe unterschiedlich starke Filterplatten für Röntgenverfahren hergestellt werden sollen. Die Kamera besitzt zwei Objektive. Innerhalb der Kamera ist ein Blitzlichtprojektor angeordnet, der mittels eines ebenfalls innerhalb der Kamera befindlichen Gitters ein Linienmuster auf den zu photographierenden Gegenstandes projiziert. Das Bild des Gegenstandes mit dem Linienmuster gelangt durch das zweite Objektiv auf das Filmmaterial. Unmittelbar vor dem Filmmaterial befindet sich ein dem genannten Gitter entsprechendes Gitter, dessen Abbildung auf dem Filmmaterial erzeugt wird. Der Vergleich der zwei unterschiedlichen Linienmuster ermöglicht einen Eindruck von der Oberflächenform des Gegenstandes. Eine Projektion des Gitters auf das Filmmaterial mit Hilfe eines Projektors erfolgt hier nicht.

Der vorliegenden Erfindung liegt von daher die Aufgabe zugrunde, eine Vorrichtung der eingangs erwähnten Art anzugeben, die auch in relativ schmalen Räumen untergebracht werden kann, d. h. die keine großen Anforderungen an Räumlichkeiten stellt und die wesentlich unempfindlicher gegen Veränderungen der Feineinstellung und gegen unerwünschten Lichteinfall ist.

Diese Aufgabe wird dadurch gelöst, daß der Projektor und das Diapositiv innerhalb der Kamera angeordnet sind und daß der Spiegel zwischen dem Objektiv der Kamera und der Bild- bzw. Filmebene angeordnet ist. Durch die erfindungsgemäße Anordnung läßt sich eine kompakte Bauweise erzielen, die unempfindlich ist gegen ungewollte Veränderungen der Feineinstellung und unerwünschten Lichteinfall vermeidet. Der lichtdurchlässige Spiegel projiziert das vergrößerte Bild des Maßrasters auf die Bildebene, ohne daß das durch das Objektiv in die Kamera einfallende Bild des Gegenstandes von dem Spiegel reflektiert wird.

Zur Justierung des Projektors ist es von Vorteil, wenn in der Objektebene, d. h. neben dem Gegenstand, eine vertikale Meßlatte mit Markierungen angeordnet ist, auf die der Projektor und das Maßraster ausgerichtet werden. Für die Ausrichtung wird zweckmäßigerweise anstelle des Filmmaterials in der Bildebene eine Mattscheibe angeordnet. Da bei der Ermittlung der menschlichen Körpermaße das Verhältnis von Höhe zu Breite sehr groß ist, hat es sich als vorteilhaft erwiesen, das vorhandene Filmmaterial zweifach auszunutzen, indem das Filmmaterial nach der Belichtung der ersten Hälfte seitlich verschoben wird. Bei einer Abmessung des Filmmaterials von 7 × 10 cm beträgt die Größe des Bildes 3,5 × 10 cm. Man kann dann, wie es bei der Vermessung von Körpern häufig sinnvoll ist, auf der einen Hälfte eine Frontansicht und auf der anderen Hälfte eine Seitenansicht darstellen.

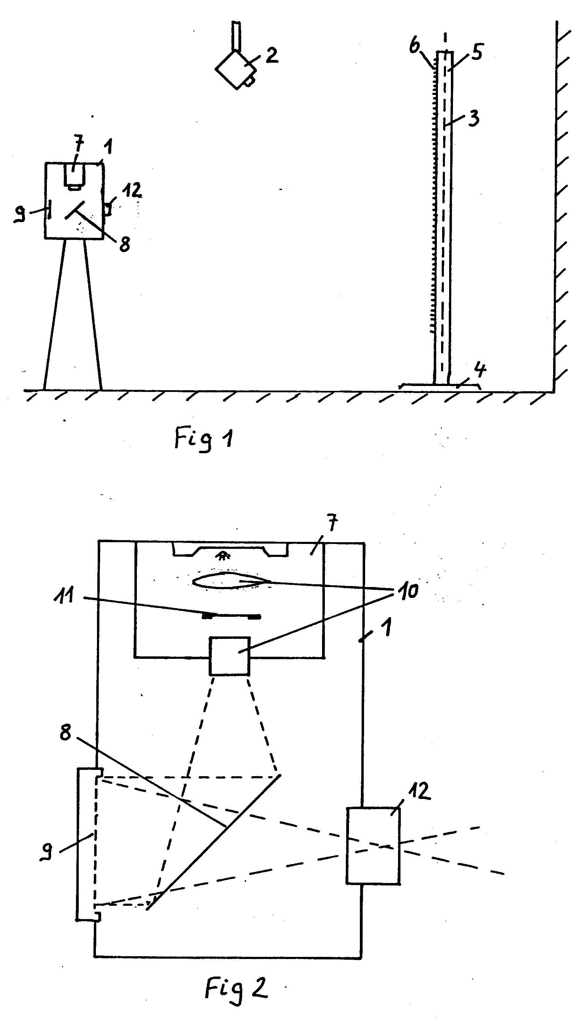

Die Erfindung ist anhand der in den Figuren 1 bis 2 schematisch dargestellten Ausführungsbeispiele näher erläutert.

In der Figur 1 ist eine seitliche Ansicht einer Vorrichtung gemäß der Lehre der Erfindung dargestellt, in der mit 1 eine Kamera, beispielsweise eine Sofortbildkamera bezeichnet ist. Oberhalb der Kamera 1 ist ein Blitzprojektor 2, der wie in der DE-PS 29 48 010 näher beschrieben ein Linienmuster aus horizontalen Linien während der Aufnahme auf den darzustellenden Gegenstand 3 projiziert. Mit 4 ist eine Balanceplatte bezeichnet, die bei der Vermessung eines Menschen anstelle des Gegenstandes 3 die Normalstellung des Menschen während der Aufnahme gewährleisten soll. Die Balanceplatte ist in der DE-OS 33 01 864 näher beschrieben. Mit 5 ist eine neben dem Gegenstand 3 befindliche Meßlatte bezeichnet, die Markierungen 6 zeigt, die zur Justierung der Anordnung dienen soll, wobei anstelle des Filmmaterials eine Mattscheibe verwendet wird. Zur Darstellung eines Maßrasters auf der herzustellenden Photographie dient ein Projektor 7, der wie weiter unten näher beschrieben über einen Spiegel 8 das Maßraster auf den Film 9 projiziert. Der Spiegel 8 ist halbdurchlässig, d.h. das von dem Projektor 7 projizierte Bild des Maßrasters wird auf den Film 9 reflektiert, wogegen das Bild des Gegenstandes 3 von dem Spiegel 8 nahezu ungehindert durchgelassen wird. Hierzu verwendet man eine mit einer dünnen Quecksilberschicht bedampfte Glasplatte.

Die Figur 2 zeigt die Kamera 1 in gegenüber der Figur 1 vergrößerter Darstellung. Der Projektor 7 weist ein optisches System 10 auf, in welches ein das Maßraster zeigendes Diapositiv 11 einschiebbar ist. Das Maßraster ist auf dem geschwärzten Diapositiv 11 durch ausgesparte horizontale und vertikale Linien gebildet. Das Bild des Maßrasters wird von dem halbdurchlässigen Spiegel 8 auf das Filmmaterial 9 reflektiert. Gleichzeitig tritt durch das Objektiv 12 der Kamera 1 das Bild des Gegenstandes 3 ein, welches durch den Spiegel 8 auf das Filmmaterial 9 projiziert wird.

Die Erfindung wurde am Beispiel einer Photokamera beschrieben, läßt sich aber mit den gleichen Vorteilen bei einer Videokamera einsetzen.

Ein Beispiel für diesen letztgenannten Fall ist die Vermessung von größeren Flächen, wobei die Videokamera in konstantem Abstand zur zu vermessenden Fläche bewegt wird, und gleichzeitig ein gebündelter Lichtstrahl, vorzugsweise ein Laser schräg von oben, vorzugsweise unter einem Winkel von 45° auf die Fläche gerichtet wird, der gemeinsam mit der Videokamera bewegt wird. Das Maßraster wird wie oben beschrieben mittels einer Dauerlichtquelle auf den Videofilm projiziert.

Ein bevorzugtes Beispiel dieses Meßverfahrens könnte die Vermessung der Innenkontur von Schmelzöfen sein, wobei dann die Videokamera mit dem Laser um die zentrale Achse des Ofens gedreht und nach jeder Umdrehung in Achsrichtung um einen bestimmten Wert verschoben wird.

## Patentansprüche

1. Vorrichtung zur Ermittlung der Abmessungen eines Gegenstandes auf photographischem Wege, insbesondere der menschlichen Körpermaße, bei der mit einer Kamera (1) eine Photographie des Gegenstandes zusammen mit einem Maßraster herstellbar ist und während der Aufnahme ein schräg von oben einfallendes Muster aus horizontal verlaufenden Linien auf den Gegenstand projizierbar ist, bei der das Maßraster mittels eines Projektors (7) und eines das Maßraster aufweisenden Diapositivs (11) direkt auf die Bildebene bzw. den Film (9) projizierbar ist, bei der ein unter einem Winkel von nahezu 45° zur Bild- bzw. Filmebene (9) geneigter lichtdurchlässiger Spiegel (8) vorgesehen ist und bei der der Projektor (7) auf den Spiegel (8) ausgerichtet ist, dadurch gekennzeichnet, daß der Projektor (7) und das Diapositiv (11) innerhalb der Kamera (1) angeordnet sind, und daß der Spiegel (8) zwischen dem Objektiv (12) der Kamera (1) und der Bildbzw. Filmebene (9) angeordnet ist.

## Claims

1. Device for photographically determining the dimensions of an object, especially the dimensions of the human body, in which a photograph of the object together with a dimensional raster can be produced by a camera (1) and during the exposure a pattern which is incident obliquely from above and which consists of horizontally extending lines can be projected onto the object, in which the dimensional raster can be projected directly onto the image plane or the film (9) by means of a projector (7) and a slide (11) exhibiting the dimensional raster, and in which a light-transmitting mirror (8) inclined at an angle of approximately 45° to the image plane or film plane (9) is provided, and in which the projector (7) is aligned onto the mirror (8), characterised in that the projector (7) and the slide (11) are disposed within the camera (1), and in that the mirror (8) is disposed between the lens (12) of the camera (1) and the image plane or film plane (9).

## Revendications

1. Dispositif pour déterminer les dimensions d'un sujet par voie photographique, notamment des dimensions du corps humain, dispositif selon lequel une photographie du sujet ainsi qu'une trame de mesure sont réalisées à l'aide d'une caméra (1) et pendant la prise de vue, un tracé de lignes horizontales est projeté en biais par le haut sur le sujet, la diapositive (11) comportant la trame de mesure étant projetée directement par un projecteur (7) dans le plan image ou plan du film (9), dispositif selon lequel un miroir transparent (8) est incliné sous un angle voisin de 45° par rapport au plan image ou plan du film (9) et le projecteur (7) est dirigé sur le miroir (8), dispositif caractérisé en ce que le projecteur (7) et la diapositive (11) sont prévus dans la caméra (1) et en ce que le miroir (8) est prévu entre l'objectif (12) de la caméra (1) et le plan image ou plan de film (9).

Fig 1

Fig 2